(19) Europäisches Patentamt
European Patent Office
Office européen des brevets

(11) **EP 4 523 599 A1**

(12) **EUROPÄISCHE PATENTANMELDUNG**

(43) Veröffentlichungstag:
**19.03.2025 Patentblatt 2025/12**

(21) Anmeldenummer: **24190278.2**

(22) Anmeldetag: **23.07.2024**

(51) Internationale Patentklassifikation (IPC):
**A61B** (2006.01)

(52) Gemeinsame Patentklassifikation (CPC):
**A61B 5/14552;** A61B 2560/0209

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **27.07.2023 DE 102023119940**

(71) Anmelder: **Löwenstein Medical Technology S.A.
2557 Luxembourg (LU)**

(72) Erfinder: **Samie, Farzad
76137 Karlsruhe (DE)**

(74) Vertreter: **Löwenstein Medical IP
Löwenstein Medical Technology
GmbH + Co.KG IP Management
Kronsaalsweg 40
22525 Hamburg (DE)**

Bemerkungen:
Geänderte Patentansprüche gemäss Regel 137(2) EPÜ.

(54) **STEUERUNG EINER LICHTQUELLE EINES PULSOXYMETERS**

(57) Ein Verfahren zum Steuern einer Lichtquelle (3, 3a, 3b) eines Pulsoxymeters (1) umfasst: Empfangen eines ersten Eingabewerts (17), der eine elektrische Größe in Bezug auf die Lichtquelle anzeigt, wobei die elektrische Größe einen durch die Lichtquelle fließenden elektrischen Strom und/oder eine an der Lichtquelle anliegende elektrische Spannung umfasst; Empfangen eines zweiten Eingabewerts (19), der eine Amplitude eines von einem Lichtsensor (5) des Pulsoxymeters erzeugten Sensorsignals (11) anzeigt; Bestimmen mindestens eines Grenzwerts für die elektrische Größe unter Verwendung des zweiten Eingabewerts, wobei der mindestens eine Grenzwert dem kleinsten Sollwert (t) oder dem größten Sollwert ($t + \varepsilon$) eines Sollwertbereichs (21), in dem die Amplitude liegen soll, zugeordnet ist; Bestimmen mindestens eines Abweichungswerts ($\Delta$), der eine Abweichung des ersten Eingabewerts vom mindestens einen Grenzwert anzeigt; Erzeugen eines Steuersignals (23) zum Steuern der Lichtquelle unter Verwendung des mindestens einen Abweichungswerts, sodass sich die Amplitude dem Sollwertbereich annähert.

Fig. 1

EP 4 523 599 A1

## Beschreibung

### Technisches Gebiet

Die Erfindung betrifft ein Verfahren zum Steuern einer Lichtquelle eines Pulsoxymeters. Des Weiteren betrifft die Erfindung eine Steuereinheit, ein Computerprogramm und ein computerlesbares Medium zum Ausführen des Verfahrens sowie ein Pulsoxymeter.

### Stand der Technik

Ein Pulsoxymeter ist im Allgemeinen eine Vorrichtung zur nicht invasiven Ermittlung der arteriellen Sauerstoffsättigung über die Messung der Lichtabsorption oder Lichtremission beim Durchleuchten von durchblutetem Körpergewebe. Ein solches Pulsoxymeter kann auch zur Pulsfrequenzkontrolle verwendet werden.

Pulsoxymeter werden zunehmend in tragbaren, batteriebetriebenen Anwendungen eingesetzt. So kann ein Pulsoxymeter beispielsweise während eines Notfalltransports an einem Patienten angebracht werden und bei der Verlegung zwischen verschiedenen Krankenhausabteilungen bei ihm verbleiben. Darüber hinaus können Pulsoxymeter als Plug-in-Module für Multiparameter-Patientenmonitore mit begrenztem Energiebudget eingesetzt werden. Solche Anwendungen führen zu einer steigenden Nachfrage nach Pulsoxymetern mit geringerem Stromverbrauch.

### Offenbarung der Erfindung

Eine Aufgabe der Erfindung kann darin gesehen werden, ein Verfahren zu schaffen, mit dem der Stromverbrauch eines Pulsoxymeters, insbesondere eines batteriebetriebenen Pulsoxymeters, ohne nennenswerte Leistungseinbußen verringert werden kann. Eine weitere Aufgabe der Erfindung kann darin gesehen werden, eine Steuereinheit, ein Computerprogramm und ein computerlesbares Medium zum Ausführen eines solchen Verfahrens sowie ein entsprechendes Pulsoxymeter bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Verfahren zum Steuern einer (elektrischen) Lichtquelle eines Pulsoxymeters. Das Pulsoxymeter umfasst ferner einen Lichtsensor, der ausgebildet ist, um einen von einem Körperteil beim Bestrahlen mit Licht von der Lichtquelle transmittierten und/oder reflektierten Lichtanteil in ein (elektrisches) Sensorsignal umzuwandeln. Das Verfahren umfasst: Empfangen eines ersten Eingabewerts, der eine elektrische Größe in Bezug auf die Lichtquelle anzeigt, wobei die elektrische Größe einen durch die Lichtquelle fließenden elektrischen Strom und/oder eine an der Lichtquelle anliegende elektrische Spannung umfasst; Empfangen eines zweiten Eingabewerts, der eine Amplitude des Sensorsignals anzeigt; Bestimmen mindestens eines Grenzwerts für die elektrische Größe unter Verwendung des zweiten Eingabewerts, wobei der mindestens eine Grenzwert dem kleinsten oder größten Sollwert eines Sollwertbereichs, in dem die Amplitude liegen soll, zugeordnet ist; Bestimmen mindestens eines Abweichungswerts, der eine Abweichung des ersten Eingabewerts vom mindestens einen Grenzwert anzeigt; Erzeugen eines Steuersignals zum Steuern der Lichtquelle unter Verwendung des mindestens einen Abweichungswerts, sodass sich die Amplitude dem Sollwertbereich annähert.

Das Verfahren ermöglicht es, im Betrieb des Pulsoxymeters Strom zu sparen, indem beispielsweise der durch die Lichtquelle fließende Strom, nachstehend Lichtquellenstrom genannt, einerseits verringert wird, solange die Qualität des Photoplethysmogramms (kurz PPG) aus dem Sensorsignal noch ausreichend ist und die erforderliche Genauigkeit noch gegeben ist, und andererseits erhöht wird, falls stärkeres Rauschen auftritt oder allgemein die Qualität des Sensorsignals schlechter wird.

Um eine ausreichende Qualität bei der Bestimmung der Sauerstoffsättigung sicherstellen zu können, sollte der pulsatile Anteil des Sensorsignals im PPG, auch AC-Anteil genannt, einen bestimmten Wert nicht unterschreiten.

Im Allgemeinen verhält sich die Amplitude des Sensorsignals bei jedem Patienten annähernd linear zum Lichtquellenstrom. Dies liegt daran, dass sich physiologische Faktoren, die die Lichtabsorption von durchblutetem Körpergewebe beeinflussen, wie etwa Fingerdicke oder Hautfarbe, während einer Messung nicht maßgeblich ändern.

Um den Sollwert zu erreichen, kann der Lichtquellenstrom im Betrieb entsprechend angepasst werden. Um zu starke und/oder zu häufige Anpassungen zu vermeiden, sollte jedoch ein Sollwertbereich für die Amplitude definiert werden. Solange der AC-Anteil im Sollwertbereich liegt, ist keine Anpassung des Lichtquellenstroms erforderlich. Liegt der AC-Anteil unter dem kleinsten Sollwert des Sollwertbereichs, so wird der Lichtquellenstrom entsprechend erhöht. Liegt der AC-Anteil hingegen über dem größten Sollwert des Sollwertbereichs, so wird der Lichtquellenstrom entsprechend verringert.

Das Verfahren kann computerimplementiert sein.

Unter "Sensorsignal" kann ein analoges oder digitales Signal verstanden werden. Unter "Amplitude" kann insbesondere eine Amplitude des AC-Anteils des Sensorsignals verstanden werden.

Unter "elektrischer Größe" kann auch eine auf dem Strom und/oder der Spannung basierende elektrische Größe und/oder eine den Strom und/oder die Spannung beeinflussende elektrische Größe verstanden werden. Beispielsweise kann es sich bei der elektrischen Größe auch um mindestens eine der folgenden Größen handeln: einen der Lichtquelle vorgeschalteten einstellbaren Vorwiderstand; eine Frequenz, mit der die Lichtquelle ein- und ausgeschaltet wird; ein zeitliches Verhältnis zwischen Einschaltphasen, in denen die Lichtquelle eingeschaltet ist, und Ausschaltphasen, in denen die Lichtquelle ausgeschaltet ist. Beispielsweise kann es sich bei der Frequenz um eine Taktfrequenz und/oder bei dem zeitlichen Verhältnis um einen Tastgrad (englisch *duty cycle*) im Kontext einer Pulsweitenmodulation der Lichtquelle handeln.

Unter "Sollwert" wie in "kleinster Sollwert" oder "größter Sollwert" kann eine vorbestimmte Konstante verstanden werden. Es ist jedoch auch möglich, dass der kleinste oder größte Sollwert im Betrieb variiert wird oder beide Sollwerte im Betrieb variiert werden, beispielsweise abhängig von einer (gemessenen oder geschätzten) Änderung bestimmter physiologischer Faktoren des jeweiligen Patienten.

Ein zweiter Aspekt der Erfindung betrifft eine Steuereinheit. Die Steuereinheit umfasst Mittel, die konfiguriert sind, um das vor- und nachstehend beschriebene Verfahren auszuführen.

Die Mittel können Hard- und/oder Softwaremodule umfassen. Insbesondere können die Mittel einen Prozessor umfassen, der konfiguriert ist, um das (computerimplementierte) Verfahren auszuführen. Zusätzlich können die Mittel einen Speicher und/oder eine Datenkommunikationsschnittstelle zur drahtlosen und/oder drahtgebundenen Datenkommunikation mit Peripheriegeräten, beispielsweise einem Smartphone, einer Smartwatch, einem Tablet, einem Laptop, einem PC oder einem Beatmungsgerät, umfassen. Alternativ kann die Steuereinheit ausschließlich als Hardware, beispielsweise in Form eines ASIC-Bausteins (ASIC = *application-specific integrated circuit*) oder FPGA-Bausteins (FPGA = *field-programmable gate array*), implementiert sein.

Es wird darauf hingewiesen, dass Merkmale des vor- und nachstehend beschriebenen Verfahrens auch Merkmale der Steuereinheit sein können (und umgekehrt).

Ein dritter Aspekt der Erfindung betrifft ein Pulsoxymeter. Das Pulsoxymeter umfasst eine Lichtquelle, einen Lichtsensor und eine Steuereinheit, wie sie vor- und nachstehend beschrieben wird. Der Lichtsensor ist ausgebildet, um einen von einem Körperteil beim Bestrahlen mit Licht von der Lichtquelle transmittierten und/oder reflektierten Lichtanteil in ein (elektrisches) Sensorsignal umzuwandeln. Das Pulsoxymeter, beispielsweise dessen Steuereinheit, kann ausgebildet sein, um eine Sauerstoffsättigung ($sO_2$) und/oder einen Puls aus dem Sensorsignal durch Absorptionsspektroskopie zu bestimmen.

Unter "Lichtquelle" kann beispielsweise eine Leuchtdiode, eine Laserdiode, eine Glühlampe oder eine Kombination aus mindestens zwei dieser Beispiele verstanden werden. Insbesondere kann die Lichtquelle ausgebildet sein, um Licht in mindestens zwei voneinander abweichenden vorbestimmten Wellenlängenbereichen, beispielsweise Rotlicht, (Nah-)Infrarotlicht oder Grünlicht, auszusenden. Beispiele für geeignete Wellenlängenbereiche sind 660 nm, 750 nm bis 850 nm und 905 nm bis 940 nm.

Unter "Lichtsensor" kann beispielsweise eine Fotodiode, eine Fotozelle, ein CMOS-Sensor (CMOS = *complementary metal-oxide-semiconductor*), ein CCD-Sensor (CCD = *charge-coupled device*) oder eine Kombination aus mindestens zwei dieser Beispiele verstanden werden.

Unter "Pulsoxymeter" kann auch ein CO-Oxymeter verstanden werden.

Das Pulsoxymeter kann beispielsweise als Clip zur Befestigung am Körperteil, beispielsweise einem Finger, einem Ohrläppchen oder einem Handgelenk, ausgebildet sein.

Das Pulsoxymeter kann so ausgebildet sein, dass die Lichtquelle und der Lichtsensor auf der gleichen Seite des Körperteils oder auf einander gegenüberliegenden Seiten des Körperteils angeordnet werden können.

Weitere Aspekte der Erfindung betreffen ein Computerprogramm und ein computerlesbares Medium, auf dem das Computerprogramm gespeichert ist.

Das Computerprogramm umfasst Befehle, die einen Prozessor - beispielsweise einen Prozessor der vor- und nachstehend beschriebenen Steuereinheit - beim Ausführen des Computerprogramms durch den Prozessor veranlassen, das vor- und nachstehend beschriebene Verfahren auszuführen.

Das computerlesbare Medium kann ein flüchtiger oder nicht flüchtiger Datenspeicher sein. Beispielsweise kann das computerlesbare Medium eine Festplatte, ein USB-Speichergerät (USB = *universal serial bus*), ein RAM *(random-access memory),* ein ROM *(read-only memory),* ein EPROM *(erasable programmable read-only memory),* ein EEPROM *(electrically erasable programmable read-only memory),* ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale des vor- und nachstehend beschriebenen Verfahrens auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann der mindestens eine Grenzwert durch Multiplizieren des ersten Eingabewerts mit einem Quotienten aus dem kleinsten oder größten Sollwert und dem zweiten Eingabewert bestimmt werden. Anders ausgedrückt kann sich der mindestens eine Grenzwert im Betrieb abhängig von einer Änderung des ersten Eingabewerts, d. h. der elektrischen Größe in Bezug auf die Lichtquelle, bzw. des zweiten Eingabewerts, d. h. der Amplitude des Sensorsignals, ändern.

Gemäß einer Ausführungsform kann der mindestens eine Abweichungswert durch Subtrahieren des ersten Eingabewerts vom mindestens einen Grenzwert bestimmt werden.

Gemäß einer Ausführungsform kann der mindestens eine Grenzwert einen dem kleinsten Sollwert zugeordneten unteren Grenzwert umfassen. In diesem Fall kann ein erster Abweichungswert durch Subtrahieren des ersten Eingabewerts vom unteren Grenzwert bestimmt werden. Das Steuersignal kann dann unter Verwendung des ersten Abweichungswerts erzeugt werden.

Gemäß einer Ausführungsform kann der unter Grenzwert $L_{2m}$ bestimmt werden gemäß

$$L_{2m} = \frac{t}{A_1} \times L_1,$$

wobei $t$ für den kleinsten Sollwert, $L_1$ für den ersten Eingabewert und $A_1$ für den zweiten Eingabewert steht. Bei dem unteren Grenzwert kann es sich beispielsweise um einen Wert der elektrischen Größe handeln, bei dem die Lichtquelle, beispielsweise deren Helligkeit und/oder Farbe, so eingestellt ist, dass die Amplitude des Sensorsignals (annähernd) mit dem kleinsten Sollwert übereinstimmt.

Gemäß einer Ausführungsform kann der mindestens eine Grenzwert einen dem größten Sollwert zugeordneten oberen Grenzwert umfassen. In diesem Fall kann ein zweiter Abweichungswert durch Subtrahieren des ersten Eingabewerts vom oberen Grenzwert bestimmt werden. Das Steuersignal kann dann unter Verwendung des zweiten Abweichungswerts erzeugt werden. Bei dem oberen Grenzwert kann es sich beispielsweise um einen Wert der elektrischen Größe handeln, bei dem die Lichtquelle, beispielsweise deren Helligkeit und/oder Farbe, so eingestellt ist, dass die Amplitude des Sensorsignals (annähernd) mit dem größten Sollwert übereinstimmt.

Gemäß einer Ausführungsform kann der obere Grenzwert $L_{2M}$ bestimmt werden gemäß

$$L_{2M} = \frac{t+\varepsilon}{A_1} \times L_1,$$

wobei $t + \varepsilon$ für den größten Sollwert (hier für eine Summe aus dem kleinsten Sollwert $t$ und einem vorbestimmten Abstand $\varepsilon$ zwischen dem kleinsten und dem größten Sollwert), $L_1$ für den ersten Eingabewert und $A_1$ für den zweiten Eingabewert steht.

Gemäß einer Ausführungsform können der erste Abweichungswert und der zweite Abweichungswert, insbesondere deren Vorzeichen, miteinander verglichen werden. In diesem Fall kann das Steuersignal nur dann erzeugt werden oder der Durchschnittswert nur dann bestimmt werden, wenn das Vorzeichen des ersten Abweichungswerts mit dem Vorzeichen des zweiten Abweichungswerts übereinstimmt. Somit kann mit geringem Rechenaufwand festgestellt werden, ob der erste Eingabewert, beispielsweise der aktuelle Lichtquellenstrom, innerhalb oder außerhalb eines durch den unteren und den oberen Grenzwert festgelegten Grenzwertbereichs liegt, was aufgrund des (annähernd) linearen Zusammenhangs zwischen dem aktuellen Lichtquellenstrom und der aktuellen Amplitude des Sensorsignals wiederum einen Rückschluss darauf zulässt, ob die aktuelle Amplitude innerhalb oder außerhalb des Sollwertbereichs liegt. Liegt sie innerhalb des Sollwertbereichs, so ist keine Anpassung des Lichtquellenstroms erforderlich.

Gemäß einer Ausführungsform kann ein Durchschnittswert aus dem ersten Abweichungswert und dem zweiten Abweichungswert bestimmt werden. Das Steuersignal kann dann unter Verwendung des Durchschnittswerts als des mindestens einen Abweichungswerts erzeugt werden. Der Durchschnittswert kann beispielsweise ein arithmetischer, geometrischer oder quadratischer Mittelwert sein. Dies kann etwaige nachfolgende Rechenschritte vereinfachen.

Gemäß einer Ausführungsform kann ein Anpassungswert unter Verwendung des mindestens einen Abweichungswerts und einer Zuordnungsvorschrift, die möglichen Abweichungswerten jeweils einen Anpassungswert zuordnet, bestimmt werden. In diesem Fall kann ein Ausgabewert unter Verwendung des ersten Eingabewerts und des Anpassungswerts, insbesondere durch Addieren des ersten Eingabewerts und des (positiven oder negativen) Anpassungswerts, bestimmt werden. Das Steuersignal kann dann unter Verwendung des Ausgabewerts erzeugt werden. Unter "Zuordnungsvorschrift" kann beispielsweise eine mathematische Funktion oder eine Lookup-Tabelle verstanden werden. Die Zuordnungsvorschrift kann beispielsweise in einem Speicher der vor- und nachstehend beschriebenen Steuereinheit hinterlegt sein.

Gemäß einer Ausführungsform kann die Zuordnungsvorschrift eine Sigmoidfunktion sein oder auf einer Sigmoidfunktion basieren. Unter "Sigmoidfunktion" kann im Allgemeinen eine Funktion mit einem S-förmigen Graphen verstanden werden. Die Sigmoidfunktion kann einen linearen oder annähernd linearen Abschnitt umfassen. Ein solcher Abschnitt kann unter anderem für die Stabilität des Verfahrens relevant sein.

Gemäß einer Ausführungsform kann die Zuordnungsvorschrift folgendermaßen definiert sein:

$$f(\Delta) = \frac{2 \times S}{1+2^{-\Delta}} - S,$$

wobei $f(\Delta)$ für den Anpassungswert, $\Delta$ für den mindestens einen Abweichungswert und S für einen größten zulässigen (im Betrieb des Pulsoxymeters variierbaren oder konstanten) Betrag des Anpassungswerts oder eine Schrittgröße steht.

Gemäß einer Ausführungsform kann eine Approximation P für den Term $2^{-\Delta}$ basierend auf einer Reihenentwicklung, bevorzugt einer Taylorreihe, besonders bevorzugt einer maclaurinschen Reihe, bestimmt werden. In diesem Fall kann die Zuordnungsvorschrift folgendermaßen definiert sein:

$$f(\Delta) = \frac{2 \times S}{1+P} - S.$$

Dies ermöglicht eine verbesserte Recheneffizienz gegenüber Ausführungsformen, wo der Term $2^{-\Delta}$ statt P berechnet wird. Somit kann der Stromverbrauch des Pulsoxymeters weiter verringert werden. Zudem erleichtert dies die Implementierung des Verfahrens als Hard- und/oder Software.

Gemäß einer Ausführungsform kann die Approximation P folgendermaßen definiert sein:

$$\text{wenn } -\Delta \geq 0, \text{ dann } P = 1 + \sum_{n=1}^{N} \frac{\left(k \times (-\Delta)\right)^n}{n!}$$

und/oder

$$\text{wenn } -\Delta < 0, \text{ dann } P = \frac{1}{1+\sum_{n=1}^{N} \frac{\left(-k \times (-\Delta)\right)^n}{n!}}.$$

Dabei steht $N$ für eine vorbestimmte Ordnung der Reihenentwicklung und $k$ für einen vorbestimmten Faktor. Beispielsweise kann $N$ eine natürliche Zahl zwischen 1 und 10, bevorzugt 5, sein und/oder $k$ ein Prozentwert zwischen 0 und 1, bevorzugt zwischen 0,5 und 1,0, besonders bevorzugt 0,75, sein.

Dies ermöglicht eine besonders recheneffiziente und/oder besonders einfach zu implementierende Approximation, ohne dass die Genauigkeit des Verfahrens merklich beeinträchtigt wird.

Gemäß einer Ausführungsform kann ein Abstand zwischen dem kleinsten und dem größten Sollwert bis zu 10 Nanoampere, bevorzugt bis zu 5 Nanoampere, besonders bevorzugt bis zu 1 Nanoampere, betragen.

Gemäß einer Ausführungsform kann der kleinste Sollwert zwischen 1 und 5 Nanoampere, bevorzugt zwischen 1 und 3 Nanoampere, besonders bevorzugt bei 2 Nanoampere, liegen.

Mit solchen Stromwerten konnte in der Praxis ein guter Kompromiss zwischen Genauigkeit und Energieeffizienz erzielt werden.

Dabei kann der Lichtquellenstrom theoretisch in einem Bereich zwischen 3 mA und 50 mA liegen. In der Praxis kann der Lichtquellenstrom abhängig vom jeweiligen Patienten auch Werte außerhalb dieses Bereichs annehmen.

Gemäß einer Ausführungsform kann das Pulsoxymeter ferner eine Batterie zur Stromversorgung mindestens einer elektrischen oder elektronischen Komponente des Pulsoxymeters, insbesondere der Lichtquelle, oder des gesamten Pulsoxymeters umfassen. Dies ermöglicht es, das Pulsoxymeter auch unterwegs zu tragen.

Gemäß einer Ausführungsform kann das Pulsoxymeter ferner eine Anzeigeeinheit zum Anzeigen mindestens eines unter Verwendung des Sensorsignals bestimmten Werts, beispielsweise einer Sauerstoffsättigung oder eines Pulses, umfassen.

## Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.

Fig. 1 zeigt ein Pulsoxymeter gemäß einer Ausführungsform der Erfindung.

Fig. 2 zeigt ein Diagramm, das einen Sollwertbereich zur Verwendung in einem Verfahren gemäß einer Ausführungsform der Erfindung veranschaulicht.

Fig. 3 zeigt ein Diagramm, das eine Zuordnungsvorschrift zur Verwendung in einem Verfahren gemäß einer Ausführungsform der Erfindung veranschaulicht.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

## Ausführungsformen der Erfindung

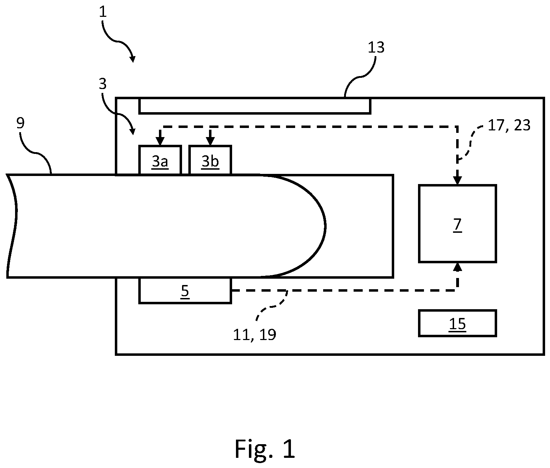

Fig. 1 zeigt ein Pulsoxymeter 1, das eine (elektrische) Lichtquelle 3, einen Lichtsensor 5 und eine Steuereinheit 7 umfasst. Der Lichtsensor 5 ist ausgebildet, um einen von einem Körperteil 9, beispielsweise einem Finger, einem Ohrläppchen oder einem ähnlich dünnen Körperteil, beim Bestrahlen mit Licht von der Lichtquelle 3 transmittierten und/oder reflektierten Lichtanteil in ein (elektrisches) Sensorsignal 11 umzuwandeln.

In diesem Beispiel ist die Steuereinheit 7 ausgebildet, um eine Sauerstoffsättigung ($sO_2$) und/oder einen Puls aus dem Sensorsignal 11 zu bestimmen.

Zusätzlich kann das Pulsoxymeter 1 eine Anzeigeeinheit 13 zum Anzeigen mindestens eines Wertes und/oder mindestens einer Grafik in Bezug auf das Sensorsignal 11, insbesondere in Bezug auf die Sauerstoffsättigung und/oder den Puls, umfassen. Die Anzeigeeinheit 13 kann beispielsweise in und/oder an einem Gehäuse des Pulsoxymeters 1 angeordnet sein.

Möglich ist auch, dass das Pulsoxymeter 1 eine Batterie 15 zur Stromversorgung des Pulsoxymeters 1 umfasst. Somit kann das Pulsoxymeter 1 auch unterwegs getragen werden.

In diesem Beispiel umfasst die Lichtquelle 3 eine erste Leuchtdiode 3a zum Aussenden von Licht in einem ersten Wellenlängenbereich, beispielsweise 660 nm, und eine zweite Leuchtdiode 3b zum Aussenden von Licht in einem vom ersten Wellenlängenbereich abweichenden zweiten Wellenlängenbereich, beispielsweise 905 nm bis 940 nm. Die Steuereinheit 7 kann ausgebildet sein, um die Leuchtdioden 3a, 3b im Betrieb des Pulsoxymeters 1 einander abwechselnd ein- und auszuschalten. Die Lichtquelle 3 kann auch nur eine Leuchtdiode mit geeignet variierbarem Wellenlängenbereich umfassen. Möglich sind auch andere Typen von Lichtquellen wie Laserdioden oder Glühlampen.

Der Lichtsensor 5 kann beispielsweise eine Fotodiode, eine Fotozelle, einen CMOS-Sensor, einen CCD-Sensor oder eine Kombination aus mindestens zwei dieser Beispiele umfassen.

Das Pulsoxymeter 1 kann beispielsweise als Clip zur Befestigung am Körperteil 9 und/oder als CO-Oxymeter ausgebildet sein.

In diesem Beispiel ist das Pulsoxymeter 1 so ausgebildet, dass die Lichtquelle 3 und der Lichtsensor 5 im Betrieb des Pulsoxymeters 1 auf einander gegenüberliegenden Seiten des Körperteils 9 angeordnet sind. Der Lichtsensor 5 empfängt somit überwiegend den vom Körperteil 9 transmittierten Lichtanteil, wenn die Lichtquelle 3 den Körperteil 9 beleuchtet.

Alternativ kann das Pulsoxymeter 1 so ausgebildet sein, dass die Lichtquelle 3 und der Lichtsensor 5 im Betrieb des Pulsoxymeters 1 auf der gleichen Seite des Körperteils 9 angeordnet sind.

Die Steuereinheit 7 umfasst Mittel, die konfiguriert sind, um ein spezielles Verfahren zum Steuern einer Stromversorgung der Lichtquelle 3 auszuführen, wie es nachstehend näher beschrieben wird. Die Mittel können Hard- und/oder Softwaremodule umfassen. Insbesondere können die Mittel einen Speicher und einen Prozessor umfassen. Auf dem Speicher kann ein Computerprogramm gespeichert sein, wobei der Prozessor konfiguriert sein kann, um das Verfahren durch Ausführen des Computerprogramms auszuführen. Zusätzlich können die Mittel eine Datenkommunikationsschnittstelle zur drahtlosen und/oder drahtgebundenen Datenkommunikation mit Peripheriegeräten, beispielsweise einem Smartphone, einer Smartwatch, einem Tablet, einem Laptop, einem PC oder einem Beatmungsgerät, umfassen.

Die Steuereinheit 7 kann auch ausschließlich als Hardware, beispielsweise in Form eines ASIC- oder FPGA-Bausteins, implementiert sein.

Das Verfahren umfasst die folgenden Schritte.

In einem ersten Schritt werden in der Steuereinheit 7, beispielsweise in einem entsprechenden Hard- und/oder Softwaremodul der Steuereinheit 7, ein erster Eingabewert 17, der eine elektrische Größe in Bezug auf die Lichtquelle 3 anzeigt, und ein zweiter Eingabewert 19, der eine aktuelle Amplitude des Sensorsignals 11 anzeigt, empfangen. Bei den beiden Eingabewerten 17,19 kann es sich um gemessene und/oder geschätzte (d. h. berechnete) Werte handeln. Die elektrische Größe umfasst einen aktuell durch die Lichtquelle 3 fließenden elektrischen Strom und/oder eine aktuell an der Lichtquelle 3 anliegende elektrische Spannung. Die elektrische Größe kann auch eine auf dem Strom und/oder der Spannung basierende elektrische Größe und/oder eine den Strom und/oder die Spannung beeinflussende elektrische Größe sein. Beispielsweise kann es sich bei der elektrischen Größe auch um mindestens eine der folgenden Größen handeln: einen der Lichtquelle 3 vorgeschalteten einstellbaren Vorwiderstand; eine Frequenz, mit der die Lichtquelle 3 ein- und ausgeschaltet wird; ein zeitliches Verhältnis zwischen Einschaltphasen, in denen die Lichtquelle 3 eingeschaltet ist, und Ausschaltphasen, in denen die Lichtquelle 3 ausgeschaltet ist. Beispielsweise kann es sich bei der Frequenz um eine Taktfrequenz und/oder bei dem zeitlichen Verhältnis um einen Tastgrad im Kontext einer Pulsweitenmodulation der Lichtquelle 3 handeln. Die Amplitude kann insbesondere eine Amplitude eines AC-Anteils des Sensorsignals 11 sein.

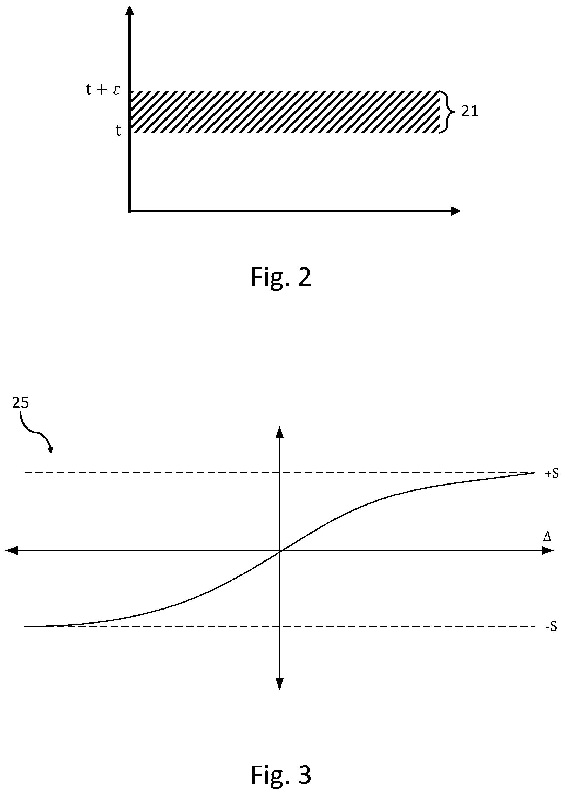

In einem zweiten Schritt wird mindestens ein Grenzwert für die elektrische Größe unter Verwendung des zweiten Eingabewerts 19, d. h. abhängig von der aktuellen Amplitude des Sensorsignals 11, bestimmt. Der mindestens eine Grenzwert ist dem kleinsten Sollwert $t$ oder dem größten Sollwert $t + \varepsilon$ eines Sollwertbereichs 21, in dem die Amplitude liegen soll, zugeordnet (siehe Fig. 2).

In einem dritten Schritt wird mindestens ein Abweichungswert, der eine Abweichung des ersten Eingabewerts 17 vom mindestens einen Grenzwert anzeigt, bestimmt.

In einem vierten Schritt wird ein Steuersignal 23 zum Steuern der Lichtquelle 3 unter Verwendung des mindestens einen Abweichungswerts erzeugt, sodass sich die Amplitude dem Sollwertbereich 21 annähert.

Das Verfahren ermöglicht es, im Betrieb des Pulsoxymeters 1 Strom zu sparen, indem beispielsweise der durch die Lichtquelle 3 fließende Strom einerseits verringert wird, solange die Qualität des Photoplethysmogramms (kurz PPG) aus dem Sensorsignal 11 noch ausreichend ist und die erforderliche Genauigkeit noch gegeben ist, und andererseits erhöht wird, falls stärkeres Rauschen auftritt oder allgemein die Qualität des Sensorsignals 11 schlechter wird.

Ein Abstand zwischen dem kleinsten Sollwert $t$ und dem größten Sollwert $t + \varepsilon$ kann beispielsweise bis zu 10 Nanoampere, bevorzugt bis zu 5 Nanoampere, besonders bevorzugt bis zu 1 Nanoampere, betragen. Zusätzlich oder alternativ kann der kleinste Sollwert $t$ zwischen 1 und 5 Nanoampere, bevorzugt zwischen 1 und 3 Nanoampere, besonders bevorzugt bei 2 Nanoampere, liegen. Solche Stromwerte erwiesen sich in der Praxis hinsichtlich der erzielten Genauigkeit und der erzielten Energieeffizienz als besonders geeignet.

Es ist möglich, dass ein unter Grenzwert $L_{2_m}$ und ein oberer Grenzwert $L_{2_M}$ für die elektrische Größe bestimmt werden. In diesem Fall kann ein erster Abweichungswert $\Delta_1$ durch Subtrahieren des ersten Eingabewerts 17 vom unteren Grenzwert $L_{2_m}$ bzw. ein zweiter Abweichungswert $\Delta_2$ durch Subtrahieren des ersten Eingabewerts 17 vom oberen Grenzwert $L_{2_M}$ bestimmt werden. Das Steuersignal 23 kann dann unter Verwendung der beiden Abweichungswerte $\Delta_1$, $\Delta_2$ erzeugt werden.

Beispielsweise kann der unter Grenzwert $L_{2_m}$ bestimmt werden gemäß

$$L_{2_m} = \frac{t}{A_1} \times L_1.$$

Zusätzlich oder alternativ kann der obere Grenzwert $L_{2_M}$ bestimmt werden gemäß

$$L_{2_M} = \frac{t + \varepsilon}{A_1} \times L_1.$$

Dabei steht $L_1$ für den ersten Eingabewert 17, hier für einen durch die jeweilige Leuchtdiode 3a bzw. 3b fließenden elektrischen Strom, und $A_1$ für den zweiten Eingabewert 19.

Dementsprechend sind die beiden Abweichungswerte $\Delta_1$, $\Delta_2$ definiert als

$$\Delta_1 = \left(\frac{t}{A_1} - 1\right) \times L_1 \text{ bzw. } \Delta_2 = \left(\frac{t + \epsilon}{A_1} - 1\right) \times L_1.$$

Zusätzlich kann aus den beiden Abweichungswerten $\Delta_1$, $\Delta_2$ ein Durchschnittswert $\Delta$ bestimmt werden, beispielsweise als arithmetischer Mittelwert gemäß

$$\Delta = \frac{\Delta_1 + \Delta_2}{2}.$$

Der Durchschnittswert $\Delta$ kann auch ein geometrischer oder quadratischer Mittelwert sein.

Das Steuersignal 23 kann dann unter Verwendung des Durchschnittswerts $\Delta$ erzeugt werden.

Beispielsweise können die Vorzeichen der beiden Abweichungswerte $\Delta_1$, $\Delta_2$ miteinander verglichen werden. In diesem Fall wird der Durchschnittswert $\Delta$ nur dann bestimmt, wenn die Vorzeichen miteinander übereinstimmen. Somit kann mit geringem Rechenaufwand festgestellt werden, ob der erste Eingabewert 17, beispielsweise der aktuelle Lichtquellenstrom, innerhalb oder außerhalb eines durch die beiden Grenzwerte $L_{2_m}$, $L_{2_M}$ festgelegten Grenzwertbereichs liegt. Dies lässt aufgrund des (annähernd) linearen Zusammenhangs zwischen dem aktuellen Lichtquellenstrom und der aktuellen Amplitude des Sensorsignals 11 wiederum einen Rückschluss darauf zu, ob die aktuelle Amplitude innerhalb oder außerhalb des Sollwertbereichs 21 liegt. Liegt sie innerhalb des Sollwertbereichs 21, so ist keine Anpassung des Lichtquellenstroms erforderlich.

Es ist möglich, dass ein Anpassungswert unter Verwendung des Durchschnittswerts $\Delta$ und einer Zuordnungs-vorschrift 25 (siehe Fig. 3), die möglichen Abweichungswerten jeweils einen Anpassungswert zuordnet, bestimmt wird. Aus dem Anpassungswert und dem ersten Eingabewert 17 kann dann ein Ausgabewert bestimmt werden, beispielsweise durch Addieren des ersten Eingabewerts 17 und des (positiven oder negativen) Anpassungswerts. Der Ausgabewert kann schließlich verwendet werden, um das Steuersignal 23 zu erzeugen.

Die Zuordnungsvorschrift 25 kann beispielsweise in Form einer mathematischen Funktion oder einer Lookup-Tabelle im Speicher der Steuereinheit 7 hinterlegt sein.

Insbesondere kann es sich bei der Zuordnungsvorschrift 25 um eine spezielle Sigmoidfunktion handeln. Die Sigmoidfunktion kann folgendermaßen definiert sein:

$$f(\Delta) = \frac{2 \times S}{1 + 2^{-\Delta}} - S,$$

wobei $f(\Delta)$ für den Anpassungswert und $S$ für einen größten zulässigen (im Betrieb des Pulsoxymeters 1 variierbaren oder konstanten) Betrag des Anpassungswerts oder eine Schrittgröße steht. Dadurch können unerwünschte Schwankungen und/oder Sprünge bei der Anpassung der Amplitude vermieden werden.

Um den Rechenaufwand, insbesondere bei der Verwendung eines Mikrocontrollers, zu verringern, kann als Option eine Approximation P statt des Terms $2^{-\Delta}$ in der Sigmoidfunktion berechnet werden, sodass diese lautet:

$$f(\Delta) = \frac{2 \times S}{1 + P} - S.$$

Die Approximation wird beispielsweise entsprechend der folgenden Gleichung durchgeführt:

$$2^{-\Delta} \approx apprx(-\Delta) = \begin{cases} 1 + \sum_{n=1}^{N} \frac{\left(k \times (-\Delta)\right)^n}{n!}, & -\Delta \geq 0 \\ \dfrac{1}{1 + \sum_{n=1}^{N} \frac{\left(-k \times (-\Delta)\right)^n}{n!}}, & -\Delta < 0 \end{cases}$$

Dabei steht $N$ für eine vorbestimmte Ordnung der Reihenentwicklung und $k$ für einen vorbestimmten Faktor. Beispielsweise kann N eine natürliche Zahl zwischen 1 und 10, bevorzugt 5, sein und/oder $k$ ein Prozentwert zwischen 0 und 1, bevorzugt zwischen 0,5 und 1,0, besonders bevorzugt 0,75, sein. Die Zahl N beeinflusst die Genauigkeit der Approximation und den Rechenaufwand. Mit $N = 5$ kann ein guter Kompromiss zwischen Genauigkeit und Rechenaufwand erzielt werden.

Eine solche Reihenentwicklung kann auch als maclaurinsche Reihe bezeichnet werden. Dies ermöglicht eine besonders recheneffiziente und/oder besonders einfach zu implementierende Approximation, ohne dass die Genauigkeit des Verfahrens merklich beeinträchtigt wird.

Wie in Fig. 3 zu erkennen, umfasst die Zuordnungsvorschrift 25 einen längeren (annähernd) linearen Abschnitt um den Nullpunkt, was für die Stabilität der Berechnung wichtig ist.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugzeichen in den Ansprüchen sind nicht als Beschränkung des Umfangs des durch die Ansprüche definierten Gegenstands zu verstehen.

**Liste der Bezugszeichen**

1      Pulsoxymeter
3      Lichtquelle

| 3a | erste Leuchtdiode |
|----|----|
| 3b | zweite Leuchtdiode |
| 5 | Lichtsensor |
| 7 | Steuereinheit |
| 9 | Körperteil |
| 11 | Sensorsignal |
| 13 | Anzeigeeinheit |
| 15 | Batterie |
| 17 | erster Eingabewert |
| 19 | zweiter Eingabewert |
| 21 | Sollwertbereich |
| 23 | Steuersignal |
| 25 | Zuordnungsvorschrift |
| S | größter zulässiger Betrag des Anpassungswerts oder der Schrittgröße |
| $t$ | kleinster Sollwert |
| $t + \varepsilon$ | größter Sollwert |
| $\Delta$ | Abweichungswert, Durchschnittswert |

**Patentansprüche**

1. Verfahren zum Steuern einer Lichtquelle (3, 3a, 3b) eines Pulsoxymeters (1), wobei das Pulsoxymeter (1) ferner einen Lichtsensor (5) umfasst, der ausgebildet ist, um einen von einem Körperteil (9) beim Bestrahlen mit Licht von der Lichtquelle (3, 3a, 3b) transmittierten und/oder reflektierten Lichtanteil in ein Sensorsignal (11) umzuwandeln, wobei das Verfahren umfasst:

   Empfangen eines ersten Eingabewerts (17), der eine elektrische Größe in Bezug auf die Lichtquelle (3, 3a, 3b) anzeigt, wobei die elektrische Größe einen durch die Lichtquelle (3, 3a, 3b) fließenden elektrischen Strom und/oder eine an der Lichtquelle (3, 3a, 3b) anliegende elektrische Spannung umfasst;
   Empfangen eines zweiten Eingabewerts (19), der eine Amplitude des Sensorsignals (11) anzeigt;
   Bestimmen mindestens eines Grenzwerts für die elektrische Größe unter Verwendung des zweiten Eingabewerts (19), wobei der mindestens eine Grenzwert dem kleinsten Sollwert ($t$) oder dem größten Sollwert ($t + \varepsilon$) eines Sollwertbereichs (21), in dem die Amplitude liegen soll, zugeordnet ist;
   Bestimmen mindestens eines Abweichungswerts ($\Delta$), der eine Abweichung des ersten Eingabewerts (17) vom mindestens einen Grenzwert anzeigt;
   Erzeugen eines Steuersignals (23) zum Steuern der Lichtquelle (3, 3a, 3b) unter Verwendung des mindestens einen Abweichungswerts ($\Delta$), sodass sich die Amplitude dem Sollwertbereich (21) annähert.

2. Verfahren nach Anspruch 1,

   wobei der mindestens eine Grenzwert durch Multiplizieren des ersten Eingabewerts (17) mit einem Quotienten aus dem kleinsten Sollwert ($t$) oder dem größten Sollwert ($t + \varepsilon$) und dem zweiten Eingabewert (19) bestimmt wird; und/oder
   wobei der mindestens eine Abweichungswert durch Subtrahieren des ersten Eingabewerts (17) vom mindestens einen Grenzwert bestimmt wird.

3. Verfahren nach einem der vorhergehenden Ansprüche,

   wobei der mindestens eine Grenzwert einen dem kleinsten Sollwert ($t$) zugeordneten unteren Grenzwert umfasst, wobei ein erster Abweichungswert durch Subtrahieren des ersten Eingabewerts (17) vom unteren Grenzwert bestimmt wird, wobei das Steuersignal (23) unter Verwendung des ersten Abweichungswerts erzeugt wird; und/oder
   wobei der mindestens eine Grenzwert einen dem größten Sollwert ($t + \varepsilon$) zugeordneten oberen Grenzwert umfasst, wobei ein zweiter Abweichungswert durch Subtrahieren des ersten Eingabewerts (17) vom oberen Grenzwert bestimmt wird, wobei das Steuersignal (23) unter Verwendung des zweiten Abweichungswerts erzeugt wird.

4. Verfahren nach Anspruch 3,
   wobei der erste Abweichungswert und der zweite Abweichungswert miteinander verglichen werden und das Steuersignal (23) nur dann erzeugt wird, wenn das Vorzeichen des ersten Abweichungswerts mit dem Vorzeichen des zweiten Abweichungswerts übereinstimmt.

5. Verfahren nach Anspruch 3 oder 4,
   wobei ein Durchschnittswert ($\Delta$) aus dem ersten Abweichungswert und dem zweiten Abweichungswert bestimmt wird und das Steuersignal (23) unter Verwendung des Durchschnittswerts ($\Delta$) als des mindestens einen Abweichungswerts ($\Delta$) erzeugt wird.

6. Verfahren nach einem der vorhergehenden Ansprüche,

   wobei ein Anpassungswert unter Verwendung des mindestens einen Abweichungswerts ($\Delta$) und einer Zuordnungsvorschrift (25), die möglichen Abweichungswerten jeweils einen Anpassungswert zuordnet, bestimmt wird;
   wobei ein Ausgabewert unter Verwendung des ersten Eingabewerts (17) und des Anpassungswerts, insbesondere durch Addieren des ersten Eingabewerts (17) und des Anpassungswerts, bestimmt wird;
   wobei das Steuersignal (23) unter Verwendung des Ausgabewerts erzeugt wird.

7. Verfahren nach Anspruch 6,
   wobei die Zuordnungsvorschrift (25) eine Sigmoidfunktion ist oder auf einer Sigmoidfunktion basiert.

8. Verfahren nach Anspruch 6 oder 7,

   wobei die Zuordnungsvorschrift (25) folgendermaßen definiert ist:

   $$f(\Delta) = \frac{2 \times S}{1 + 2^{-\Delta}} - S,$$

   wobei $f(\Delta)$ für den Anpassungswert, $\Delta$ für den mindestens einen Abweichungswert ($\Delta$) und $S$ für einen größten zulässigen Betrag ($S$) des Anpassungswerts steht.

9. Verfahren nach Anspruch 8,
   wobei eine Approximation P für den Term $2^{-\Delta}$ basierend auf einer Reihenentwicklung, bevorzugt einer Taylorreihe, besonders bevorzugt einer maclaurinschen Reihe, bestimmt wird und die Zuordnungsvorschrift (25) folgendermaßen definiert ist:

   $$f(\Delta) = \frac{2 \times S}{1 + P} - S.$$

10. Verfahren nach Anspruch 9,

    wobei die Approximation $P$ folgendermaßen bestimmt wird:

    $$\text{wenn } -\Delta \geq 0, \text{ dann } P = 1 + \sum_{n=1}^{N} \frac{(k \times (-\Delta))^n}{n!}$$

    und/oder

    $$\text{wenn } -\Delta < 0, \text{ dann } P = \frac{1}{1 + \sum_{n=1}^{N} \frac{(-k \times (-\Delta))^n}{n!}};$$

    wobei $N$ für eine vorbestimmte Ordnung der Reihenentwicklung und $k$ für einen vorbestimmten Faktor steht.

11. Verfahren nach einem der vorhergehenden Ansprüche,

    wobei ein Abstand zwischen dem kleinsten Sollwert ($t$) und dem größten Sollwert ($t + \varepsilon$) bis zu 10 Nanoampere, bevorzugt bis zu 5 Nanoampere, besonders bevorzugt bis zu 1 Nanoampere, beträgt; und/oder

wobei der kleinste Sollwert ($t$) zwischen 1 und 5 Nanoampere, bevorzugt zwischen 1 und 3 Nanoampere, besonders bevorzugt bei 2 Nanoampere, liegt.

**12.** Steuereinheit (7), umfassend Mittel, die konfiguriert sind, um das Verfahren nach einem der vorhergehenden Ansprüche auszuführen.

**13.** Pulsoxymeter (1), umfassend:

eine Lichtquelle (3, 3a, 3b);
einen Lichtsensor (5), der ausgebildet ist, um einen von einem Körperteil (9) beim Bestrahlen mit Licht von der Lichtquelle (3, 3a, 3b) transmittierten und/oder reflektierten Lichtanteil in ein Sensorsignal (11) umzuwandeln;
eine Steuereinheit (7) nach Anspruch 12.

**14.** Computerprogramm, umfassend Befehle, die einen Prozessor beim Ausführen des Computerprogramms durch den Prozessor veranlassen, das Verfahren nach einem der Ansprüche 1 bis 11 auszuführen.

**15.** Computerlesbares Medium, auf dem das Computerprogramm nach Anspruch 14 gespeichert ist.

**Geänderte Patentansprüche gemäss Regel 137(2) EPÜ.**

**1.** Verfahren zum Steuern einer Lichtquelle (3, 3a, 3b) eines Pulsoxymeters (1), wobei das Pulsoxymeter (1) ferner einen Lichtsensor (5) umfasst, der ausgebildet ist, um einen von einem Körperteil (9) beim Bestrahlen mit Licht von der Lichtquelle (3, 3a, 3b) transmittierten und/oder reflektierten Lichtanteil in ein Sensorsignal (11) umzuwandeln, wobei das Verfahren umfasst:

Empfangen eines ersten Eingabewerts (17), der eine elektrische Größe in Bezug auf die Lichtquelle (3, 3a, 3b) anzeigt, wobei die elektrische Größe einen durch die Lichtquelle (3, 3a, 3b) fließenden elektrischen Strom und/oder eine an der Lichtquelle (3, 3a, 3b) anliegende elektrische Spannung umfasst;
Empfangen eines zweiten Eingabewerts (19), der eine Amplitude des Sensorsignals (11) anzeigt;
Bestimmen mindestens eines Grenzwerts für die elektrische Größe unter Verwendung des zweiten Eingabewerts (19), wobei der mindestens eine Grenzwert dem kleinsten Sollwert ($t$) oder dem größten Sollwert ($t + \varepsilon$) eines Sollwertbereichs (21), in dem die Amplitude liegen soll, zugeordnet ist;
Bestimmen mindestens eines Abweichungswerts ($\Delta$), der eine Abweichung des ersten Eingabewerts (17) vom mindestens einen Grenzwert anzeigt;
Erzeugen eines Steuersignals (23) zum Steuern der Lichtquelle (3, 3a, 3b) unter Verwendung des mindestens einen Abweichungswerts ($\Delta$), sodass sich die Amplitude dem Sollwertbereich (21) annähert, wobei ein Anpassungswert unter Verwendung des mindestens einen Abweichungswerts ($\Delta$) und einer Zuordnungsvorschrift (25), die möglichen Abweichungswerten jeweils einen Anpassungswert zuordnet, bestimmt wird, wobei die Zuordnungsvorschrift (25) eine Sigmoidfunktion ist oder auf einer Sigmoidfunktion basiert, wobei ein Ausgabewert unter Verwendung des ersten Eingabewerts (17) und des Anpassungswerts bestimmt wird, wobei das Steuersignal (23) unter Verwendung des Ausgabewerts erzeugt wird.

**2.** Verfahren nach Anspruch 1,

wobei der mindestens eine Grenzwert durch Multiplizieren des ersten Eingabewerts (17) mit einem Quotienten aus dem kleinsten Sollwert ($t$) oder dem größten Sollwert ($t + \varepsilon$) und dem zweiten Eingabewert (19) bestimmt wird; und/oder
wobei der mindestens eine Abweichungswert durch Subtrahieren des ersten Eingabewerts (17) vom mindestens einen Grenzwert bestimmt wird.

**3.** Verfahren nach einem der vorhergehenden Ansprüche,

wobei der mindestens eine Grenzwert einen dem kleinsten Sollwert ($t$) zugeordneten unteren Grenzwert umfasst, wobei ein erster Abweichungswert durch Subtrahieren des ersten Eingabewerts (17) vom unteren Grenzwert bestimmt wird, wobei das Steuersignal (23) unter Verwendung des ersten Abweichungswerts erzeugt wird; und/oder
wobei der mindestens eine Grenzwert einen dem größten Sollwert ($t + \varepsilon$) zugeordneten oberen Grenzwert umfasst, wobei ein zweiter Abweichungswert durch Subtrahieren des ersten Eingabewerts (17) vom oberen Grenzwert bestimmt wird, wobei das Steuersignal (23) unter Verwendung des zweiten Abweichungswerts erzeugt wird.

4. Verfahren nach Anspruch 3,
   wobei der erste Abweichungswert und der zweite Abweichungswert miteinander verglichen werden und das Steuersignal (23) nur dann erzeugt wird, wenn das Vorzeichen des ersten Abweichungswerts mit dem Vorzeichen des zweiten Abweichungswerts übereinstimmt.

5. Verfahren nach Anspruch 3 oder 4,
   wobei ein Durchschnittswert ($\Delta$) aus dem ersten Abweichungswert und dem zweiten Abweichungswert bestimmt wird und das Steuersignal (23) unter Verwendung des Durchschnittswerts ($\Delta$) als des mindestens einen Abweichungswerts ($\Delta$) erzeugt wird.

6. Verfahren nach einem der vorhergehenden Ansprüche,
   wobei der Ausgabewert durch Addieren des ersten Eingabewerts (17) und des Anpassungswerts bestimmt wird.

7. Verfahren nach einem der vorhergehenden Ansprüche,

   wobei die Zuordnungsvorschrift (25) folgendermaßen definiert ist:

   $$f(\Delta) = \frac{2 \times S}{1 + 2^{-\Delta}} - S,$$

   wobei $f(\Delta)$ für den Anpassungswert, $\Delta$ für den mindestens einen Abweichungswert ($\Delta$) und $S$ für einen größten zulässigen Betrag (S) des Anpassungswerts steht.

8. Verfahren nach Anspruch 7,
   wobei eine Approximation $P$ für den Term $2^{-\Delta}$ basierend auf einer Reihenentwicklung, bevorzugt einer Taylorreihe, besonders bevorzugt einer maclaurinschen Reihe, bestimmt wird und die Zuordnungsvorschrift (25) folgendermaßen definiert ist:

   $$f(\Delta) = \frac{2 \times S}{1 + P} - S.$$

9. Verfahren nach Anspruch 8,
   wobei die Approximation $P$ folgendermaßen bestimmt wird:

   $$P = 1 + \sum_{n=1}^{N} \frac{\left(k \times (-\Delta)\right)^n}{n!} \qquad P = \frac{1}{1 + \sum_{n=1}^{N} \frac{\left(-k \times (-\Delta)\right)^n}{n!}};$$

   wenn $-\Delta \geq 0$, dann und/oder wenn $-\Delta < 0$, dann
   wobei $N$ für eine vorbestimmte Ordnung der Reihenentwicklung und $k$ für einen vorbestimmten Faktor steht.

10. Verfahren nach einem der vorhergehenden Ansprüche,

    wobei ein Abstand zwischen dem kleinsten Sollwert ($t$) und dem größten Sollwert ($t + \varepsilon$) bis zu 10 Nanoampere, bevorzugt bis zu 5 Nanoampere, besonders bevorzugt bis zu 1 Nanoampere, beträgt; und/oder
    wobei der kleinste Sollwert ($t$) zwischen 1 und 5 Nanoampere, bevorzugt zwischen 1 und 3 Nanoampere, besonders bevorzugt bei 2 Nanoampere, liegt.

11. Steuereinheit (7), umfassend Mittel, die konfiguriert sind, um das Verfahren nach einem der vorhergehenden Ansprüche auszuführen.

12. Pulsoxymeter (1), umfassend:

    eine Lichtquelle (3, 3a, 3b);
    einen Lichtsensor (5), der ausgebildet ist, um einen von einem Körperteil (9) beim Bestrahlen mit Licht von der Lichtquelle (3, 3a, 3b) transmittierten und/oder reflektierten Lichtanteil in ein Sensorsignal (11) umzuwandeln;
    eine Steuereinheit (7) nach Anspruch 11.

13. Computerprogramm, umfassend Befehle, die einen Prozessor beim Ausführen des Computerprogramms durch den Prozessor veranlassen, das Verfahren nach einem der Ansprüche 1 bis 10 auszuführen.

14. Computerlesbares Medium, auf dem das Computerprogramm nach Anspruch 13 gespeichert ist.

Fig. 1

Fig. 2

Fig. 3

Europäisches Patentamt
European Patent Office
Office européen des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/054269 A1 (RANTALA BORJE [FI] ET AL) 18. März 2004 (2004-03-18) * Absätze [0015], [0049] - [0053]; Abbildungen 1,2 * | 1-7, 11-15 | INV. A61B5/14552 |
| X | US 2005/250997 A1 (TAKEDA SUNAO [JP] ET AL) 10. November 2005 (2005-11-10) * Abbildung 2 * | 1-7, 11-15 | |
| X | US 6 863 652 B2 (DRAEGER MEDICAL SYSTEMS INC [US]) 8. März 2005 (2005-03-08) * Abbildung 2 * | 1-7, 11-15 | |
| X | US 7 468 036 B1 (RULKOV NIKOLAI [US] ET AL) 23. Dezember 2008 (2008-12-23) * Abbildung 18 * | 1-7, 11-15 | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| Berlin | 18. Dezember 2024 | Clevorn, Jens |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 24 19 0278

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-12-2024

| Im Recherchenbericht angeführtes Patentdokument | Datum der Veröffentlichung | Mitglied(er) der Patentfamilie | Datum der Veröffentlichung |
|---|---|---|---|
| US 2004054269 A1 | 18-03-2004 | KEINE | |
| US 2005250997 A1 | 10-11-2005 | JP 4614047 B2<br>JP 2005278758 A<br>US 2005250997 A1 | 19-01-2011<br>13-10-2005<br>10-11-2005 |
| US 6863652 B2 | 08-03-2005 | CN 1652717 A<br>EP 1485016 A2<br>JP 2005519714 A<br>US 2004002637 A1<br>WO 03092490 A2 | 10-08-2005<br>15-12-2004<br>07-07-2005<br>01-01-2004<br>13-11-2003 |
| US 7468036 B1 | 23-12-2008 | KEINE | |

EPO FORM P0461

Für nähere Einzelheiten zu diesem Anhang : siehe Amtsblatt des Europäischen Patentamts, Nr.12/82